# FASCICULE DE BREVET EUROPEEN

(11) **EP 1 235 573 B1**
(45) Date de publication et mention de la délivrance du brevet: **23.07.2003**
(21) Numéro de dépôt: 00985315.1
(22) Date de dépôt: 24.11.2000
(51) Int. Cl.: A61K 31/425, A61P 25/00

(54) **ASSOCIATION DE RILUZOLE ET DE GABAPENTINE ET SON UTILISATION COMME MEDICAMENT DANS LE TRAITEMENT DES MALADIES MOTONEURONALES**
PHARMAZEUTISCHE ZUSAMMENSETZUNG WELCHE RILUZOL UND GABAPENTIN ENTHÄLT ZUR BEHANDUNG VON MOTONEURONALEN KRANKHEITEN
COMBINATION OF RILUZOLE AND GABAPENTIN AND USE THEREOF FOR THE TREATMENT OF MOTOR NEURON DISORDERS

(30) Priorité: 24.11.1999 FR 9914765
(43) Date de publication de la demande: 04.09.2002
(73) Titulaire: Aventis Pharma S.A., 92160 Antony (FR)
(72) Inventeur: BOHME, Andrees, F-75020 Paris (FR); HENDERSON, Christopher, F-13260 Cassis (FR)
(74) Mandataire: Rousseau, Pierrick Edouard
(86) Numéro de dépôt international: FR0003272
(87) Numéro de publication internationale: WO01037832

(56) Documents cités:
- EP-A- 0 446 570
- EP-A- 0 558 861
- LOUVEL E ET AL: "Therapeutic advances in amyotrophic lateral sclerosis" TRENDS IN PHARMACOLOGICAL SCIENCES,GB,ELSEVIER TRENDS JOURNAL, CAMBRIDGE, vol. 18, no. 6, 1 juin 1997 (1997-06-01), pages 196-203, XP004094506 ISSN: 0165-6147
- ROSS M A: "Acquired motor neuron disorders" NEUROLOGIC CLINICS,XX,XX, vol. 15, no. 3, août 1997 (1997-08), pages 481-500, XP002108929
- MITSUMOTO H. ET AL: "Drug combination treatment in patients with ALS: Current status and futur directions." NEUROLOGY, (1996) 47/4 SUPPL. (S103-S107)., XP000972165
- LAI, EUGENE C.: "Therapeutic developments in amyotrophic lateral sclerosis" EXPERT OPIN. INVEST. DRUGS (1999), 8(4), 347-361, 1999, XP000972140

## Description

La présente invention concerne l'association de riluzole et de gabapentine ou un de leurs sels pharmaceutiquement acceptables et son utilisation comme médicament utile notamment pour la prévention et/ou le traitement des maladies motoneuronales.

Les maladies motoneuronales incluent notamment la sclérose latérale amyotrophique, l'amyotrophie spinale progressive, l'amyotrophie spinale infantile et la sclérose latérale primaire. Elles résultent d'une perte progressive des motoneurones dans la moelle épinière.

La sclérose latérale amyotrophique (SLA) également connue sous le nom de maladie de CHARCOT et maladie de LOU GEHRIG a été décrite pour la première fois par CHARCOT en 1865. Elle est la maladie motoneuronale la plus importante. La SLA est une maladie mortelle résultant de la dégénérescence des motoneurones. La maladie s'accompagne d'une paralysie progressive, conduisant à la perte des fonctions motrices et respiratoires puis à la mort dans un délai de deux à huit ans après l'apparition des premiers symptômes.

A ce jour, seul le riluzole (2-amino-6-trifluorométhoxybenzothiazole) est commercialisé sous le nom de RILUTEK^{R} pour le traitement de la sclérose latérale amyotrophique. Le riluzole permet principalement de ralentir la progression de la maladie.

La gabapentine a également été préconisée pour le traitement des maladies neurodégénératives et notamment de la sclérose latérale amyotrophique (brevet US5084479). La gabapentine est actuellement commercialisée sous le nom de NEURONTIN^{R} pour le traitement des épilepsies.

Il a maintenant été trouvé que l'association de riluzole et de gabapentine ou un de leurs sels pharmaceutiquement acceptables permet d'augmenter la survie des motoneurones d'une manière plus importante que le riluzole seul ou la gabapentine seule.

L'effet de l'association a été étudié sur la mort cellulaire de motoneurones purifiés de rat induite par des sérums de patients atteints de sclérose latérale amyotrophique.

Des motoneurones purifiés préparés à partir de moelle épinière d'embryons de rat de 14 jours de gestation sont cultivés en présence de concentrations optimales de « Brain-Derived Neurotrophic Factor » (BDNF) recombinant, un des principaux facteurs neurotrophique de survie des motoneurones. 22 heures après, le riluzole et la gabapentine sont ajoutés aux motoneurones soit seuls soit en association. Après encore 2 heures, des dilutions de sérums dialysés de patients atteints de SLA sont ajoutés. Le jour suivant le nombre de motoneurones survivants est évalué par comptage direct au microscope à contraste de phase.

### Méthodes

### Composés

La solution mère de riluzole utilisée dans ce test est préparée de la manière suivante : 5 mg de riluzole sont ajoutés à 0,105 ml d'une solution d'acide chlorhydrique (1M). La solution est agitée puis on ajoute 2,1 ml d'eau bidistillée.

La solution mère de gabapentine est préparée directement par dissolution de 17,1 mg de gabapentine dans 1 ml d'eau bidistillée.

Les solutions ainsi obtenues peuvent être conservées à 4°C pendant 24 heures maximum.

Toutes les dilutions sont préparées extemporanément dans le milieu de culture complet, immédiatement avant le traitement des cellules.

### Purification des motoneurones

Des embryons de rats de 14 jours de gestation (Charles River, France) sont récupérés après sacrification de la mère, sous anesthésie au CO₂, par élongation cervicale. Ces embryons sont placés en tampon phosphate (« phosphate-buffered saline », PBS) exempt de calcium et de magnésium (Life Technologies).

Une population homogène de motoneurones purifiés est obtenue en utilisant les méthodes décrites par HENDERSON et coll [Henderson, C. E., Bloch-Gallego, E., and Camu, W. (1995). Purified embryonic motoneurons. *In* «Nerve cell culture : a practical approach» (J. Cohen and G. Wilkin, Eds.), pp. 69-81. Oxford University Press, London.] et ARCE et coll. [Arce, V., Garcès, A., de Bovis, B., Filippi, P., Henderson, C., Pettmann, B., and deLapeyrière, O. (1999). Cardiotrophin-1 requires LIFRbeta to promote survival of mouse motoneurons purified by a novel technique. J *Neurosci Res* **55**, 119-126]. Sauf mention contraire, la composition des milieux utilisés est identique à celle indiqué dans ces références. Des moelles épinières d'embryons de rat de 14 jours de gestation sont disséqués dans du PBS stérile et les moitiés ventrales de chaque moelle contenant les motoneurones sont sous-disséquées. Elles sont ensuite coupées en petits morceaux à l'aide d'un scalpel et traitées avec de la trypsine (0,25%, poids/volume) dans un milieu F10 exempt de calcium et de magnésium (Life Technologies). Après 15 minutes, la trypsine est remplacée par un milieu de culture complet L15 sans bicarbonate (commercialisé par Life Technologies, Inc.) et les neurones spinaux sont dissociés par une série de triturations de plus en plus fortes. La trypsine et les débris cellulaires sont séparés par centrifugation à travers une couche d'albumine sérique de boeuf (« bovine serum albumin », BSA) à 4%. La première étape de purification s'effectue sur un gradient de 6,8% de métrizamide. La fraction de faible densité contenant les motoneurones est centrifugée sur une couche de BSA et ensuite repurifiée par immuno-sélection à l'aide d'une colonne magnétique (Miltenyi Biotech Inc). A cette fin, les cellules sont remises en suspension dans 50 µl de PBS + 0,5 % de BSA mélangé à 50 µl de l'anticorps 192 qui reconnaît le récepteur neurotrophine de faible affinité p75^{NTR} spécifiquement exprimé par les motoneurones à ce stade de développement. Après incubation à 4°C pendant 10 minutes, les motoneurones sont centrifugés sur BSA. Les cellules sont mises à nouveau en suspension dans 80 µl de PBS + 0,5 % de BSA et 20 µl de perles magnétiques greffées en surface avec des anticorps de lapin anti-souris, puis incubées à 4°C pendant 15 minutes. Les cellules sont ensuite recentrifugées sur BSA et passées dans une colonne magnétique. Les cellules éluées sont de gros neurones; 80 à 90% de ceux-ci pouvant être montrés par immunomarquage comme exprimant la protéine Islet-1, un marqueur spécifique des motoneurones.

### Culture des motoneurones

Les motoneurones purifiés sont ensemencés à une densité de 1000 motoneurones par puits dans des puits de 16 mm préalablement enduits de polyornithine-laminine. Le milieu de culture est le milieu Neurobasal complémenté avec le complément B27 (Life Technologies) et 2 % de sérum de cheval mais sans antibiotique, le volume total étant de 0,5 ml. Les motoneurones sont ensemencés en présence de facteur neurotrophique BDNF (1ng/ml; Sigma) et laissés en sédimentation pendant 22 heures pendant lesquelles elles s'attachent au support et développement des neurites. Le riluzole (3.10⁻⁵ M) et la gabapentine (10⁻⁵M) sont ajoutés soit séparément soit en association dans des séries de 4 puits. Deux heures plus tard, un sérum dialysé de patient atteints de SLA est ajouté aux puits. Les motoneurones sont ensuite cultivés pendant 1 jour à 37,2°C avant évaluation de leur survie.

### Quantification de la survie des motoneurones

Les plaques de culture sont retirées de l'incubateur. Les puits contenant les motoneurones sont remplis complètement avec du milieu L15 (Life Technologies) tiède. Leur couvercle est ensuite remis en place de manière à former un ménisque horizontal permettant l'observation de la surface entière du milieu de culture sous optiques à contraste de phase. Pour chaque puits, le nombre total de motoneurones est compté le long de 2 diamètres orthogonaux (x et y) du champ du microscope.

Compte-tenu du fait que la présence de sérum humain change la morphologie des motoneurones mêmes survivants, toutes les cellules portant des neurites et attachées au support de culture sont considérées comme étant en vie.

### Analyse des données

Les valeurs de survie (c'est à dire le nombre de motoneurones par diamètre de puit) sont calculées comme la moyenne ± SEM de 8 mesures pour 4 puits indépendants dans chaque expérience.

Les analyses statistiques sont effectuées en utilisant le test de Student (t-test).

Les résultats obtenus sont mentionnés dans le tableau suivant :

| | | | | | |
|---|---|---|---|---|---|
| Sérum SLA | 0% | 0,1% | 0,1% | 0,1% | 0,1% |
| Riluzole (M) | 0 | 0 | 3.10⁻⁵ | 0 | 3.10⁻⁵ |
| Gabapentine (M) | 0 | 0 | 0 | 10⁻⁵ | 10⁻⁵ |
| Nombre de motoneurones | | | | | |
| (par expérience et par | 13 | 3 | 7 | 6 | 12 |
| diamètre de puits) | 26 | 4 | 9 | 5 | 19 |
| | 19 | 0 | 4 | 5 | 18 |
| | 36 | 4 | 9 | 9 | 17 |
| | 33 | 2 | 11 | 2 | 18 |
| | 33 | 4 | 7 | 9 | 14 |
| | 25 | 5 | 6 | 1 | 23 |
| | 32 | 2 | 7 | 3 | 21 |
| Moyenne | 27,1 | 3,0 | 7,5 | 5,0 | 17,8 |
| (±SEM) | ±2,8 | ±0,6 | ±0,8* | ±1,1* | ±1,3** |

| | | | | | |
|---|---|---|---|---|---|
| * p=0,0003 | | | | | |
| **p<00001 | | | | | |

Le BDNF (1ng/ml) est ajouté dans toutes les expériences comme mentionné dans la technique générale.

Ces résultats démontrent que :
a - le sérum de malade atteint de SLA induit la mort de 89% des motoneurones même en présence de support neurotrophique, laissant donc 11 % de neurones survivants.
b - lorsque le milieu est traité avec du riluzole seul, la survie des motoneurones est de 19%
c - lorsque le milieu est traité avec la gabapentine seule, la survie des motoneurones est de 8%
d - lorsque le milieu est traité avec l'association de riluzole et de gabapentine, la survie des motoneurones est de 61%, mettant ainsi en évidence une synergie entre les deux molècules.
   Le riluzole peut être préparé selon la méthode décrite dans le brevet US4370338.
   La gabapentine peut être préparée selon la méthode décrite dans les brevets FR2294697 et US4024175.
   Comme sels pharmaceutiquement acceptables du riluzole et de la gabapentine, peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.
   L'association peut être employée par voie orale, parentérale ou rectale soit simultanément soit séparément soit de manière étalée dans le temps.

La présente invention concerne également les compositions pharmaceutiques comprenant l'association de riluzole et de gabapentine ou un de leurs sels pharmaceutiquement acceptables à l'état pur ou sous forme d'une association avec un ou plusieurs diluants et/ou adjuvants compatibles et pharmaceutiquement acceptables et/ou éventuellement en association avec un autre produit pharmaceutiquement compatible et physiologiquement actif.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

La présente invention concerne également l'utilisation de l'association de riluzole et de gabapentine ou un de leurs sels pharmaceutiquement acceptables pour la préparation d'un médicament utile pour la prévention et/ou le traitement des maladies motoneuronales et, notamment, de la sclérose latérale amyotrophique, de l'amyotrophie spinale progressive, de l'amyotrophie spinale infantile ou de la sclérose latérale primaire.

La présente invention concerne également la méthode de prévention et/ou de traitement des patients atteints de maladies motoneuronales et, notamment, de la sclérose latérale amyotrophique, de l'amyotrophie spinale progressive, de l'amyotrophie spinale infantile ou de la sclérose latérale primaire qui consiste à administrer au patient une association de riluzole et de gabapentine ou un de leurs sels pharmaceutiquement acceptables soit simultanément soit séparément soit de manière étalée dans le temps.

Les doses dépendent de l'effet recherché, de la durée du traitement et de la voie d'administration utilisée; elles sont généralement de 10 à 400 mg par jour par voie orale pour un adulte avec des doses unitaires allant de 10 à 200 mg de riluzole et de 100 à 2400 mg par jour par voie orale pour un adulte avec des doses unitaires de 100 à 400 mg de gabapentine.

D'une façon générale, le médecin déterminera la posologie appropriée en fonction de l'âge, du poids et de tous les autres facteurs propres au sujet à traiter.

La présente invention concerne également une association dans laquelle on utilise 10 à 400 parties en poids de riluzole pour 300 à 2400 parties en poids de gabapentine.

## Revendications

1. Association de riluzole et de gabapentine ou un de leurs sels pharmaceutiquement acceptables.

2. Association selon la revendication 1 dans laquelle on utilise 10 à 400 parties en poids de riluzole pour 300 à 2400 parties en poids de gabapentine.

3. Association selon l'une des revendications 1 ou 2 comme préparation combinée pour une utilisation simultanée, séparée ou étalée dans le temps.

4. Composition pharmaceutique constituée d'une association selon la revendication 1 à l'état pur ou en présence de tout diluant ou adjuvant compatible et pharmaceutiquement acceptable.

5. Utilisation d'une association selon l'une des revendications 1 à 3 pour la préparation d'un médicament utile pour la prévention et/ou le traitement des maladies motoneuronales.

6. Utilisation d'une association selon l'une des revendications 1 à 3 pour la préparation d'un médicament utile pour la prévention et/ou le traitement de la sclérose latérale amyotrophique.

7. Utilisation d'une association selon l'une des revendications 1 à 3 pour la préparation d'un médicament utile pour la prévention et/ou le traitement de l'amyotrophie spinale progressive, de l'amyotrophie spinale infantile ou de la sclérose latérale primaire.

## Patentansprüche

1. Assoziation von Riluzol und Gabapentin oder einem ihrer pharmazeutisch akzeptablen Salze.

2. Assoziation nach Anspruch 1, bei der man 10 bis 40 Gewichtsteile Riluzol pro 300 bis 2400 Gewichtsteile Gabapentin verwendet.

3. Assoziation nach einem der Ansprüche 1 oder 2 als kombinierte Präparation für eine gleichzeitige, getrennte oder zeitlich ausgedehnte Anwendung.

4. Pharmazeutische Zusammensetzung, bestehend aus einer Assoziation nach Anspruch 1 in reinem Zustand oder in Anwesenheit von jedem kompatiblen und pharmazeutisch akzeptablen Verdünnungsmittel oder Zusatzstoff.

5. Verwendung einer Assoziation nach einem der Ansprüche 1 bis 3 zur Herstellung eines Arzneimittels für die Vorbeugung und/oder die Behandlung von motoneuronalen Erkrankungen.

6. Verwendung einer Assoziation nach einem der Ansprüche 1 bis 3 zur Herstellung eines Arzneimittels für die Vorbeugung und/oder die Behandlung von amyotrophischer Lateraisklerose.

7. Verwendung einer Assoziation nach einem der Ansprüche 1 bis 3 zur Herstellung eines Arzneimittels für die Vorbeugung und/oder die Behandlung von progressiver spinaler Amyotrophie, von infantiler spinaler Amyotrophie oder von primärer Lateralsklerose.

## Claims

1. Combination of riluzole and of gabapentin or one of their pharmaceutically acceptable salts.

2. Combination according to Claim 1, in which 10 to 400 parts by weight of riluzole are used per 300 to 2400 parts by weight of gabapentin.

3. Combination according to either of Claims 1 and 2, as a combined preparation for use simultaneously, separately or spaced out over time.

4. Pharmaceutical composition consisting of a combination according to Claim 1, in the pure state or in the presence of any compatible and pharmaceutically acceptable diluent or adjuvant.

5. Use of a combination according to one of Claims 1 to 3, for the preparation of a medicament useful for the prevention and/or treatment of motoneuron diseases.

6. Use of a combination according to one of Claims 1 to 3, for the preparation of a medicament useful for the prevention and/or treatment of amyotrophic lateral sclerosis.

7. Use of a combination according to one of Claims 1 to 3, for the preparation of a medicament useful for the prevention and/or treatment of progressive spinal amyotrophy, of infantile spinal amyotrophy or of primary lateral sclerosis.
